# EUROPEAN PATENT APPLICATION

(11) **EP 2 023 140 A1**
(43) Date of publication of application: **11.02.2009**
(21) Application number: 08161995.9
(22) Date of filing: 07.08.2008
(51) Int. Cl.: G01N 33/543, B01L 3/00

(54) **Biosensor chip**

(30) Priority: 08.08.2007 JP 2007206483
(71) Applicant: FUJIFILM Corporation, Minato-ku Tokyo (JP)
(72) Inventor: Minami, Koichi, Ashigarakami-gun Kanagawa-ken (JP); Takeuchi, Yohsuke, Ashigarakami-gun Kanagawa-ken (JP); Tsuzuki, Hirohiko, Ashigarakami-gun Kanagawa-ken (JP)
(74) Representative: HOFFMANN EITLE

(57) **Abstract**

[Objective]

To provide a biosensor chip which is capable of stably immobilizing bioactive substances, as well as preventing non specific adsorption of substances which are the targets of detection.

[Constitution]

A biosensor chip is constituted by: a substrate; a metal film which is physically attached to the substrate; and a self assembled monolayer constituted by alkyl chains, which is physically attached to the metal film. Ligands and at least one functional group, selected from a group consisting of a hydroxy group, a carboxyl group, an alkoxy group, a methyl group, and hydrogen atoms are physically attached to the self assembled monolayer. The ratio of the number of alkyl chains which are covalently bonded to the ligands with respect to the total number of alkyl chains on the substrate is within a range of 0.40 to 0.99.

## Description

### Background of the Invention

### Field of the Invention

The present invention relates to a biosensor chip which is capable of immobilizing bioactive substances thereon.

### Description of the Related Art

Presently, measurements that utilize intermolecular interactions, such as immune reactions in clinical tests, are being performed. Several techniques that do not require complex operations or labeling substances, and are capable of detecting variations in the bonding amounts of measurement target substances at high sensitivity, are being utilized. Examples of these techniques include: the Surface Plasmon Resonance (SPR) measurement technique, the quartz crystal microbalance (QCM) measurement techniques, and a technique that utilizes the functional surfaces of gold colloid particles to superfine particles. In all of these techniques, surfaces on which bioactive substances are immobilized are necessary. This will be explained, using Surface Plasmon Resonance (SPR) as an example.

Commonly, measurement chips which are utilized to measure bioactive substances comprise: a transparent substrate (a glass plate, for example); a metal film formed by vapor deposition on the transparent substrate; and a thin film having functional groups onto which bioactive substance such as proteins can be immobilized; stacked in this order. Bioactive substances are immobilized onto the surface of the metal film via the functional groups. Specific bonding reactions between the bioactive substances and targets of measurement are measured, to analyze the interaction among biomolecules.

There are several known techniques for immobilizing bioactive substances to measuring chips. In the case that the bioactive substance is protein, the amine coupling method is known, in which amino groups of the protein covalently bond with carboxyl groups on the measurement chip. However, in this methods, arbitrary amino groups on the surfaces of the protein are modified by the immobilization. Therefore, there are cases in which the orientation of the immobilized protein is not uniform, and cases in which the activity of the protein is decreased because bonding between the protein and the carboxyl group is hindered due to the position of the amino group. In addition, in this method, it is necessary to condense the protein on the chip. However, it is necessary for the protein to be dissolved in a low ion strength buffer liquid having a lower pH than the pI of the protein. Therefore, in the case that the protein is of a type that becomes denatured under these conditions, it is not possible for the protein to be immobilized while maintaining the activity thereof.

Meanwhile, techniques for immobilizing proteins onto measurement chips under neutral conditions by using portions called Tags, which are introduced into the N terminals or the C terminals of artificial proteins synthesized by genetic modification, are also being developed. The immobilization technique using His-tag is a representative example of such a technique. This technique was developed for affinity columns that purify His-tag proteins which are expressed by gene recombination. However, this technique is also used to immobilize proteins with a uniform orientation.

Particularly in immobilization of His-tag proteins using NTA-Ni(II) complexes formed by NTA (Nitrilotric acetic acid) and Ni (II) ions, water molecules positioned at two coordinate positions of the complexes replace the nitrogen atoms of two imidazole groups of oligohistidine residue of the His-tag proteins. Thereby, the His-tag proteins specifically bond with surfaces with a uniform orientation. It is not necessary to perform pre-concentration of the His-tag proteins under acidic conditions when the NTA-Ni(II) complexes are used to immobilize the proteins. Therefore, immobilization of the His-tag proteins is possible using buffers (such as PBS) under physiological conditions, and the problems encountered during amine coupling can be resolved.

However, the combination of the His-tag proteins and the NTA-Ni(II) complexes has been developed with the objective of purification by affinity columns. Therefore, the bonds are not sufficiently strong, and dissociative equilibria are present. Accordingly, there is a problem that His-tag proteins which have been immobilized on measurement chips via NTA-Ni(II) complexes gradually dissociate from the measurement chips, and therefore this immobilization technique is not suited for biosensor chips.

Various methods have been proposed to solve this problem of dissociation. For example, methods in which oxidizing agents are used to oxidize and deactivate the His-tag proteins are proposed in Japanese Unexamined Patent Publication Nos. 2006-266831 and 6(1994)-157600. However, if these methods are employed, there are cases in which the proteins become deactivated, depending on the oxidation speed, the oxidizing agent, and the like. A method in which triNTA is substituted for the NTA as ligands to perform immobilization in order to improve the bonding properties is proposed in International Patent Publication No. WO00/47548. However, this method cannot obtain sufficient immobilization for practical use.

Meanwhile, "Double-Hexahistidine Tag with High-Affinity Binding for Protein Immobilization, Purification, and Detection on Ni-Nitrilotriacetic Acid Surfaces", F. Khan et al., Anal. Chem., Vol. 78, pp. 3072-3079, 2006 discloses the use a polysaccharide onto which NTA is immobilized, as a thin film having functional groups for immobilizing bioactive substances such as proteins thereon. "Surface characterization and platelet compatibility evaluation of the binary mixed self-assembled monolayers", M-Y. Tsai et al., Journal of Colloid and Interface Science, Vol. 308, pp. 474-484, 2007 discloses the use of a self assembled monolayer (mixed SAM) constituted by alkyl chains having different functional groups and lengths, as a thin film having functional groups for immobilizing bioactive substances thereon. This mixed SAM is effective in preventing non specific adsorption of substances which are the targets of detection.

In measurement chips which are utilized to measure bioactive substances, it is important for non specific adsorption of targets of detection to be suppressed, in addition to stable immobilization of the bioactive substances thereon. From the disclosures of the aforementioned Anal. Chem. And Journal of Colloid and Interface Science articles, it is considered that immobilization of ligands which are coordinated at multiple points, such as NTA, on a mixed SAM would enable bioactive substances to be stably immobilized, as well as prevent non specific adsorption of substances which are the targets of detection. However, when NTA is immobilized onto a mixed SAM, there is a problem that the SAM separates from a substrate due to the bulkiness of the ligands, when metal ions for forming chains are caused to flow thereon.

### SUMMARY OF THE INVENTION

The present invention has been developed in view of the foregoing circumstances. It is an object of the present invention to provide a biosensor chip which is capable of stably immobilizing bioactive substances, as well as preventing non specific adsorption of substances which are the targets of detection.

A biosensor chip of the present invention comprises:
a substrate;
a metal film which is physically attached to the substrate; and
a self assembled monolayer constituted by alkyl chains, which is physically attached to the metal film; characterized by:
   ligands and at least one functional group, selected from a group consisting of a hydroxyl group, a carboxyl group, an alkoxy group, a methyl group, and hydrogen atoms being physically attached to the self assembled monolayer; and
   the ratio of the number of alkyl chains which are covalently bonded to the ligands with respect to the total number of alkyl chains on the substrate being within a range of 0.40 to 0.99.

It is preferable for the of the number of alkyl chains which are covalently bonded to the ligands with respect to the total number of alkyl chains on the substrate to be within a range of 0.45 to 0.85.

It is preferable for metal ions to be coordinately bonded to the ligands.

It is preferable for bioactive substances to be coordinately bonded to the metal ions.

It is preferable for the ligands to be nitrilotriacetic acid derivatives.

It is preferable for the alkyl chains that constitute the self assembled monolayer to be covalently bonded with the metal film by at least one functional group, selected from a group consisting of: -SH, -SS-, -SeH, -SeSe, and -COSH.

It is preferable for the self assembled monolayer to be formed by a compound represented by the following General Formula (1) and a mixture that contains a compound represented by the following General Formula (2).

X-R^{a}-Y (1)

X-R^{b}-Z (2)

(Here, X represents a group which is capable of covalent bonding with the metal film; R^{a} and R^{b} represent organic divalent linking groups; Y is a functional group selected from a group consisting of: a hydroxyl group, a carboxyl group, an alkoxy group, a methyl group, and hydrogen atoms; and Z represents a ligand.)

It is preferable for the carbon number of the alkyl chain length of R^{a} to be within a range from 2 to 8, and more preferably within a range from 4 to 8.

It is preferable for the difference in carbon numbers between R^{a} and R^{b} to be within a range from 2 to 6.

It is preferable for the metal ions to be Cu(II) ions.

It is preferable for the metal film to be formed by at least one metal selected from a group consisting of: gold, silver, copper, platinum, palladium, and aluminum.

The biosensor chip of the present invention comprises the metal film formed on the substrate and the self assembled monolayer formed by the alkyl chains, provided on the metal film. The ligands and the at least one functional group, selected from a group consisting of a hydroxyl group, a carboxyl group, an alkoxy group, a methyl group, and hydrogen atoms are physically attached to the self assembled monolayer. The ratio of the number of alkyl chains which are covalently bonded to the ligands with respect to the total number of alkyl chains on the substrate is within a range of 0.40 to 0.99. Therefore, it is possible to stably immobilize bioactive substances on the biosensor chip. In addition, non specific adsorption of targets to be detected can be suppressed.

That is, the ratio of the number of alkyl chains which are covalently bonded to the ligands with respect to the total number of alkyl chains on the substrate is set to the aforementioned range. Thereby, the self assembled monolayer is prevented from separating from the substrate due to the bulkiness of the ligands, even when metal ions for forming chains are caused to flow thereon. Accordingly, the bioactive stables are capable of being stably immobilized on the biosensor chip.

### BRIEF DESCRIPTION OF THE DRAWINGS

Figure 1 is a schematic diagram that illustrates the construction of a biosensor chip according to a first embodiment of the present invention.
Figure 2 is a schematic diagram that illustrates the bonded state of a self assembled monolayer formed by alkyl chains and NTA.
Figure 3 is a schematic diagram that illustrates an alternate bonded state of a self assembled monolayer formed by alkyl chains and NTA.

### BEST MODE FOR CARRYING OUT THE INVENTION

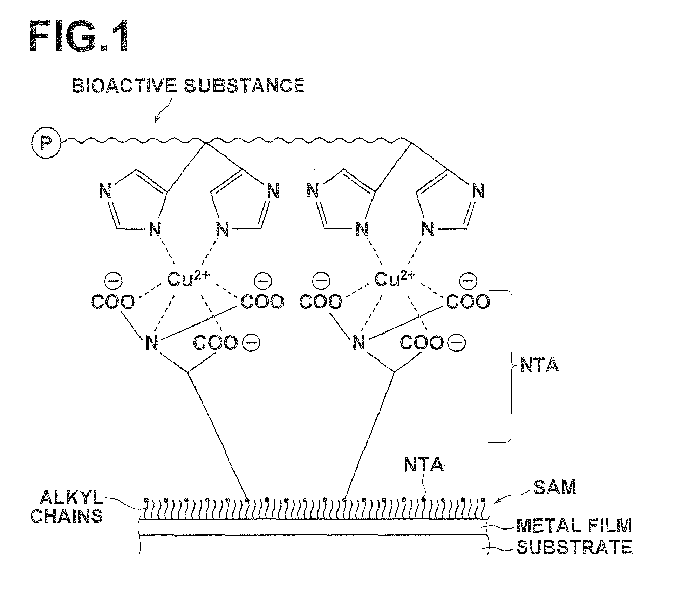

Hereinafter, the biosensor chip of the present invention will be described with reference to the attached drawings. Figure 1 is a schematic diagram that illustrates the construction of a biosensor chip according to a first embodiment of the present invention. Note that a portion of Figure 1 is magnified to clearly illustrate bonding states. NTA is employed as the ligands, Cu(II) is employed as the metal ions, and proteins having histidine units are employed as the bioactive substance. A state in which pairs of NTA's are bonded to the histidine units is illustrated in Figure 1.

The biosensor chip illustrated in Figure 1 is equipped with a sensor chip substrate, on the surface of which a metal film is provided. A self assembled monolayer (SAM) formed by alkyl chains is physically attached to the metal film. NTA is provided on the self assembled monolayer as ligands, metal ions (Cu(II)) are coordinately bonded to the NTA, and the proteins having histidine units are coordinately bonded to the metal ions. The ends of the alkyl chains of the self assembled monolayer which are not bonded to the NTA are functional groups, selected from a group consisting of a hydroxyl group, a carboxyl group, an alkoxy group, a methyl group, and hydrogen atoms.

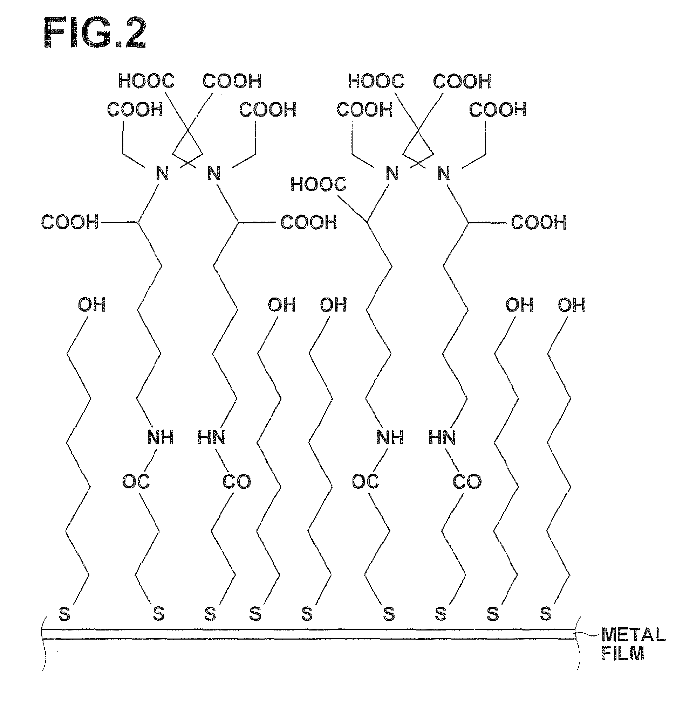

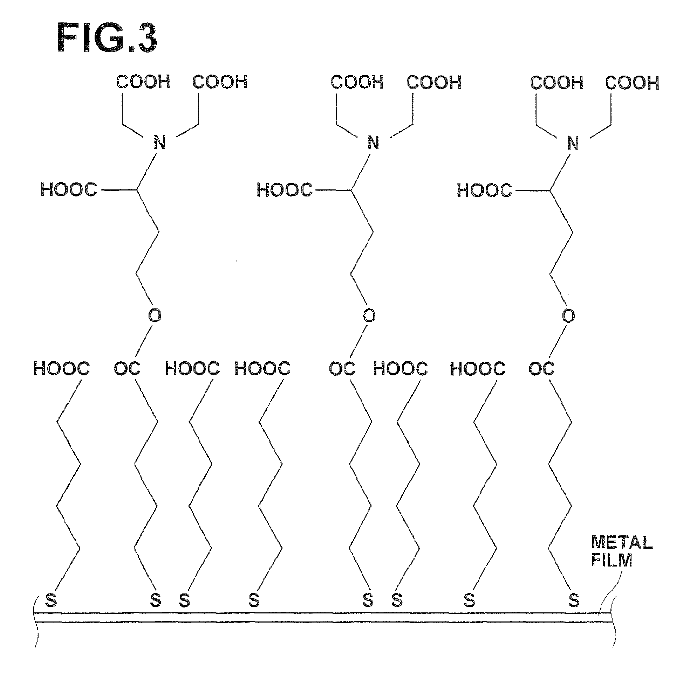

The details of the bonding state are illustrated in Figure 2 and Figure 3. Figure 2 and Figure 3 illustrate the bonded portions of the self assembled monolayer formed by alkyl chains and the NTA in detail. The alkyl chains have first ends and second ends. As illustrated in Figure 2 and Figure 3, the alkyl chains that form the self assembled monolayer are covalently bonded to the metal film on the substrate by a group which is capable of bonding with the metal film, such as -S, at the first ends thereof. The group at the first ends of the alkyl chains is at least one functional group, selected from a group consisting of: -SH, -SS-, -SeH, -SeSe, and -COSH. The NTA is bonded with the second ends (at least one functional group, selected from a group consisting of a hydroxyl group, a carboxyl group, an alkoxy group, a methyl group, and hydrogen atoms) of the alkyl chains.

Hereinafter, each of the structures of the biosensor chip of the present invention and the methods for forming (activating) the structures will be described.

### (1) Substrate and Metal Film

In the case that use in a surface plasmon resonance biosensor is considered, materials which are transparent with respect to laser beams may be utilized as the material for the substrate of the biosensor chip. Examples of such materials include: optical glass, such as BK7; and synthetic resin, such as polymethyl methacrylate, polyethylene terephthalate, polycarbonate, and cycloolefin polymer. It is desirable for the substrate to be formed by a material that does not exhibit anisotropy with respect to polarization, and is superior in workability.

The metal film is physically attached to the substrate. Here, "physically attached to the substrate" includes cases in which the metal film is physically attached to the substrate via another layer, that is, without directly contacting the substrate, in addition to cases in which the metal film is in direct contact with the substrate. The metal to be utilized as the material of the metal film is not particularly limited, as long as it is capable of generating surface plasmon resonance. However, it is preferable for the material to be at least one metal, selected from a group consisting of: gold, silver, copper, platinum, palladium, and aluminum. Gold is particularly preferred. These metals may be used singly or in combination. In addition, a layer of chrome, for example, may be interposed between the substrate and the metal film.

The thickness of the metal film may be set as desired. However, it is preferable for the thickness of the metal film to be within a range from 0.1 nm, to 500 nm, and more preferably within a range from 1 nm to 200 nm. This is because surface plasmon phenomena of media cannot be sufficiently detected if the thickness of the metal film exceeds 500 nm. In the case that the layer of chrome or the like is interposed between the substrate and the metal film, the thickness of the interposed layer is preferably within a range from 0.1 nm to 10 nm.

The metal film may be formed by known methods. Examples of such methods include: sputtering; vapor deposition; ion plating; electrolytic plating; and non-electrolytic plating.

### (2) Self Assembled Monolayer

Self assembled monolayers refer to ultrathin films, such as monomolecular films and LB films, which have uniform regularity imparted by the mechanism of the film material, without external control being exerted. Self assembled monolayers enable formation of uniformly regular structures and patterns over long distances under non equilibrial conditions.

The self assembled monolayer that constitutes the biosensor chip of the present invention is formed by the alkyl chains, having at least one functional group, selected from a group consisting of: -SH (thiol), -SS (sulfide), -SeH (selenol), -SeSe (diselenide), and -COSH (thioic acid) at the first ends thereof, and at least one functional group, selected from a group consisting of a hydroxyl group, a carboxyl group, an alkoxy group, a methyl group, and hydrogen atoms at the second ends thereof.

More preferably, the self assembled monolayer is formed by a compound represented by the following General Formula (1) or a mixture that contains a compound represented by the following General Formula (2). The compound represented by the following General Formula (1) or the mixture that contains a compound represented by the following General Formula (2) may be (i) a mixture that includes at least one compound represented by General Formula (1). More preferably, the compound represented by the following General Formula (1) or the mixture that contains a compound represented by the following General Formula (2) may be (ii) a mixture that includes at least one compound represented by General Formula (1) and at least one compound represented by General Formula (2). In the case of (i), several different types of alkyl chains having different lengths and functional groups may be included, and ligands such as NTA may be covalently bonded via the functional groups. In the case of (ii), if the ratio of compounds represented by General Formula (2) and the total number of alkyl chains is within a ratio of 0.40 to 0.99, there is no need to covalently bond with ligands, to be described later.

X-R^{a}-Y (1)

X-R^{b}-Z (2)

(X represents a group which is capable of covalent bonding with the metal film such as -SH, -SS, -SeH, and -COSH; R^{a} and R^{b} represent organic divalent linking groups; Y is a functional group selected from a group consisting of: a hydroxyl group, a carboxyl group, an alkoxy group, a methyl group, and hydrogen atoms; and Z represents a ligand.)

Here, R^{a} and R^{b}, which are organic divalent linking groups, may be interrupted by heteroatoms. Preferably, R^{a} and R^{b} are straight (non branching) chains due to dense packing. R^{a} and R^{b} may be hydrocarbon chains that include double and/or triple bonds. Carbon chains may be hyper fluorinated, if necessary. It is preferable for the carbon number of the alkyl chain length of R^{a} in General Formula (1) to be within a range from 2 to 8. It is preferable for the carbon number of the alkyl chain length of R^{b} in General Formula (2) to be within a range from 4 to 8. It is particularly preferable for the alkyl chains of R^{a} and R^{b} to be different, and for the alkyl chain of R^{b} to be longer. It is preferable for the difference in the carbon number of the alkyl chain of R^{a} and the carbon number of the alkyl chain of R^{b} to be within a range from 2 to 6, and more preferably within a range from 2 to 4. In the present invention, the carbon numbers of alkyl chains are determined by counting each heteroatom as a single carbon atom, in the case that they are present.

It is preferable for Z to be nitrilotriacetic acid, iminodiacetic acid, or derivatives thereof. Y is a functional group selected from a group consisting of a hydroxyl group, a carboxyl group, an alkoxy group, a methyl group, and hydrogen atoms. In the case that Y is a methyl group or a hydrogen atom, Y refers to the second ends of the alkyl chains. In the case that Y is an alkoxy group, it is preferable for the alkoxy group to be a methoxy group or an ethoxy group, having a carbon number from 1 to 2. Here, the term "derivatives" refers to compounds having partial structures that correspond to the structures of the groups.

In order for the alkyl chains to be physically attached to the surface of the substrate at an appropriate density, it is desirable for the alkyl chains to be straight (non branching) chains. The alkyl chains may include double and/or triple bonds as necessary. The alkyl chain lengths of all of the alkyl chains which are bonded to the substrate may be the same. However, from the viewpoint of immobilizing targets of detection efficiently onto the substrate, it is preferable for the alkyl chain lengths of the alkyl chains having the functional groups for immobilizing the targets of detection onto the self assembled monolayer to be different. (Note that Figure 2 and Figure 3 illustrate cases in which the alkyl chain lengths of the alkyl chains are different. Figure 2 illustrates a case in which alkyl chains have carbon numbers of 6 and 8, and Figure 3 illustrates a case in which alkyl chains have carbon numbers of 4 and 8.)

6-hydroxy-1-hexanethiol; 1-hexanethiol; 5-hydroxy carbonyl-1-pentanethiol; 6-methoxy-1-hexanethiol; 1-octanethiol; 8-hydroxy-1-octanethiol; 7-hydroxy carboxyl- 1-heptanethiol; 8-methoxy-1-octanethiol and 1-heptanethiol are preferred examples of specific structures that satisfy General Formula (1). 3,3'-dithio bis[N-(5-amino-5-carboxy pentyl) propion amide-N,N'-diacetyl acid]; 2,2'-dithio bis [N-(5-amino-5-carboxy pentyl) acetyl amide-N,N'-diacetyl acid]; 3-thiopropion amide-N-(5-amino-5-carboxy pentyl) propion amide-N,N' diacetyl acid are preferred examples of specific structures that satisfy General Formula 2. The above may be used singly or as appropriate combinations.

Coating methods for metal films using self assembled monolayers have been developed by Professor Whitesides of Harvard University et al. The details thereof are reported in Chemical Review, Vol. 105, pp. 1103-1169 (2005), for example. In the case that gold is used as the metal, the -S groups, which are derived from the -SH, -SS, -SeH, -SeSe, or -COSH groups at the ends of a self assembled monolayer forming compound, and Au at the surface of the gold form Au-S bonds. Thereby, a monomolecular film having uniform orientation is formed in a self assembling manner, based on van der Waal forces among the alkyl chains. The self assembled monolayer can be produced by a simple technique of immersing a gold substrate within a solution containing the self assembled monolayer forming compound.

### (3) Ligands

Various chelate solutions may be used as compounds to become the ligands. Preferred examples include polydentate ligands, such as: nitrilotriacetic acid (NTA (N-(5-amino-1-carboxypentyl) iminodiacetic acid)); iminodiacetic acid; phenanthroline; terpyridine; bipyridine; triethylene tetraamine; bi (ethylene triamine); tris (carboxy methyl) ethylene diamine; diethylene triamine pentaacetic acid; polypyrazolyl boric acid; 1,4,7-triazacyclononane; dimethyl glyoxime; diphenyl glyoxime; and derivatives thereof. Among these, nitrilotriacetic acid and derivatives thereof are preferred. Nitrilotriacetic acid is a tridentate ligand. Therefore, the ligands are converted to three neighboring carboxyl groups, by the ligands bonding with the at least one functional group selected from the group consisting of: a hydroxyl group, a carboxyl group, an alkoxy group, a methyl group, and hydrogen atoms.

The ligands can be caused to bond with the carboxyl groups, by: activating the carboxyl groups; then causing the ligands to react therewith, for example. Alternatively, the substrate may be immersed in C2-NTA represented by the following chemical formula, having NTA as functional groups at the ends thereof, and the self assembled monolayer forming compound, to cause the ligands to bond.

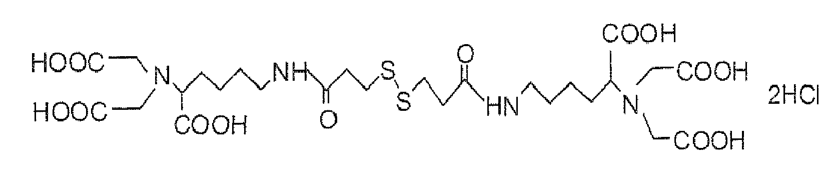

The ligands are preferably caused to bond with 40% to 99% of the alkyl chains on the substrate, and more preferably, caused to bond with 45% to 85% of the alkyl chains on the substrate. If the ligands bond with more than 99% of the alkyl chains on the substrate, the self assembled monolayer becomes too thin, and there is a possibility that the alkyl chains will separate from the substrate, when metal ions are caused to flow thereon. On the other hand, if the ligands bond with less than 40% of the alkyl chains on the substrate, the bioactive substance cannot be stably immobilized.

The ligands such as NTA can be caused to bond at the aforementioned percentages, by covalent bonding either directly or via a coupler. In the case that the self assembled monolayer is in the form of (i) described above, the chip of the present invention may be formed by bonding the ligands to the functional group Y of General Formula (1). At this time, the size (volume) of the ligands prevents bonding of the ligands to all of the functional groups. Specifically, the carboxyl groups which are present within the self assembled monolayer are activated by EDC (1-(3-dimethyl aminopropyl)-3-ethylcarbodiimide), then AB-NTA (N-(5-amino-1-carboxyl pentyl) iminodiacetic acid) having amino groups at the ends thereof is caused to act to bond the AB-NTA to the substrate by amide bonds.

Alternatively, in the case that a compound that has NTA as functional groups at the ends thereof, such as C2-NTA (that is, in the case that the self assembled monolayer is in the form of (ii) described above), an amount of this compound and an amount of the self assembled monolayer forming compound may be mixed at a ratio that will result in a desired percentage of alkyl chains being bonded to the ligands.

Note that: an acid chloride method, in which phosphorous oxychloride or phosphorous trichloride is caused to react with carboxylic acid; a phosphoric acid ester method, in which diethyl chlorophosphoric acid, diphenyl chlorophosphoric acid and the like are caused to react with carboxylic acid; an electron withdrawing group substitution by benzoate ester method, in which p-nitro benzoic acid chloride, 2,4-dinitro benzoic acid chloride and the like are caused to react with carboxylic acid; and the like may be employed to activate the carboxyl group, instead of using the aforementioned EDC.

It is preferable for the density of the ligands to be within a range from 1x10¹¹/mm³ to 1x10¹²/mm³, and more preferably within a range from 5x10¹¹/mm³ to 1x10¹²/mm³. In the case that the density of the ligands is less than 1x10¹¹/mm³, it becomes difficult to hold the bioactive substance. On the other hand, if the density of the ligands exceeds 1x10¹²/mm³, the orientation of the self assembled monolayer will be disturbed due to three dimensional obstacles, which is not favorable.

### (4) Metal Ions

Any metal ion may be employed as the metal ions, as long as they form unsaturated metallic complexes. From the viewpoint of stability of obtained metallic complexes, transitional metal ions are preferred. Specifically, appropriate metal ions may be selected from among a group consisting of: Ni(II); Ni (III) ; Ni(IV); Cu(I); Cu(II); Co (II) ; Co (III) ; Fe (II) ; and Fe(III), according to the type of ligand. Cu(II) is particularly preferred as the metal ions, because it is easy to control the bonding amounts thereof, and because Cu(II) has strong bonding properties. In the case that the metal ions are Cu(II), it is preferable for the ligand density to be 1x10¹¹/mm³ or greater. Note that the bonding force of metal ions varies according to valence. In the case of Co(II) and Fe(II), the oxidation number of metal ions can be changed by the oxidation reduction method described in paragraphs [0037] and [0038] of Japanese Unexamined Patent Publication No. 6(1994) -157600, to vary the bonding forces thereof.

### (5) Bioactive Substance

Examples of the bioactive substance include: immunoproteins; enzymes; microorganisms; nucleic acids; low molecular weight organic compounds; anti-immunoproteins; immunoglobulin binding proteins; glycobinding proteins; sugar chains that recognize sugars; fatty acids; fatty acid esters; polypeptides having ligand binding potential; and oligopeptides having ligand binding potential. These bioactive substances are immobilized on the substrate by coordinate bonding with the metal ions, and have functional groups which are capable of being coordinated with respect to the metal ions. That is, the bioactive substances have metallic coordinating properties. The metallic coordinating properties can be easily imparted, by covalent bonding with ligands having strong coordinating forces.

The functional group may be any functional group, as long as it has a nitrogenous heterocyclic ring, and is capable of forming metallic complexes with the metal ions. The nitrogenous heterocyclic ring may be a monocycle or a condensed three to seven member ring including at least one nitrogen atom. Preferably, the nitrogenous heterocyclic ring is a five member ring or a six member ring. Examples of ligands having such nitrogenous heterocyclic rings include: pyrrole; imidazole; pyrazole; oxazole; isooxazole; thiazole; isothiazole; 1,2,3-triazole; 1,2,4-triazole; 1,3,4-thiadiazole; tetrazole; pyridine; pyrazine; pyrimidine; pyridazine; 1,2,3-triazine; 1,2,4-triazine; 1,3,5-triazine; 1,2,4,5-tetrazine; azepine; azonine; quinoline; isoquinoline; acridine; phenanthridine; indole; isoindole; carbazole; benzimidazole; 1,8-naphthyridine; purine; pteridine; benzotriazole; quinoxaline; quinazoline; perimidine; cinnoline; phthaladine; 1,10-phenanthroline; phenoxazine; phenothiazine; phenazine; 8-hydroxyquinoline; 8-mercaptoquinoline; 2,2'-bipyridine; 2,2'-dipyridyl amine; di (2-picolyl amine); 2,2',2"-terpyridine porphyrin; phthalocyanine; and derivatives thereof.

Among the above ligands, pyrrole, imidazole, pyrazole, oxazole, thiazole, pyridine, and derivatives thereof are preferable, from the viewpoint of improving the stability of the obtained metallic complexes.

Particularly, imidazole groups are preferred as the functional group, due to the ease of introduction thereof using amino acid automatic synthesis systems or genetic modification. It is preferable for so called His-tags, in which histidines (His) including imidazole groups are introduced as functional sites, to be long. It is preferable for the number of imidazole groups to be within a range from 6 to 100. The hisitidines may be consecutive, such as His-His-His-His, and may also have different structures therebetween, such as His-His-(different structure)-His-His. An example in which histidines (His) having imidazole groups were introduced was described as a preferred arrangement of functional groups. However, the type of functional group is not limited to imidazole groups.

### (6) Immobilization of the Bioactive Substance

The bioactive substance is immobilized, by coating the biosensor chip with a solution that includes the bioactive substance, then drying biosensor chip. In the present invention, "coating" includes immersion. In the case that the bioactive substance includes the nitrogenous heterocyclic rings, the nitrogenous heterocyclic rings form coordinate bonds with the metal ions and form chains, thereby immobilizing the bioactive substance.

When the metal ions and the nitrogenous heterocyclic rings of the bioactive substance are added to the ligands which are bonded to the substrate, (1) the ligands, (2) the nitrogenous heterocyclic rings, and (3) water molecules or hydroxide ions are coordinated on the metal ions to form chains.

For example, in the case that NTA is utilized as the ligands, and metal ions capable of six coordinates are added, (1) three carboxyl groups and one nitrogen atom of the NTA occupy four of the six coordinate sites, (2) a nitrogenous heterocyclic ring group of the bioactive substance occupies one of the two remaining coordinate sites, and (3) a water molecule or a hydroxide ion occupies the last remaining coordinate site, to form six coordinate complexes.

In the case that iminodiacetic acid is utilized as the ligands, and metal ions capable of six coordinates are added, (1) two carboxyl groups and one nitrogen atom of the iminodiacetic acid occupy three of the six coordinate sites, and (2) nitrogenous heterocyclic ring groups of the bioactive substance and (3) water molecules or hydroxide ions occupy the remaining three coordinate sites, to form six coordinate complexes.

Here, metal ions capable of six coordinates have been described as examples. However, the number of coordinate sites may be seven or greater, or five or less. In addition, the carboxyl groups that form the complexes need not be provided by a single ligand, but from a plurality of ligands.

### (7) Application of the Biosensor Chip

The biosensor chip of the present invention may be employed in sensors that convert interactions among biological molecules into signals, such as electric signals, to measure and/or detect target substances. More specifically, the biosensor chip of the present invention may be employed in: biosensor, constituted by receptor sites that recognize chemical substances as detection targets, and transducer sites that convert physical or chemical changes at the receptor sites into electric signals. The biosensor chip of the present invention may also be employed in conjunction with the surface plasmon resonance (SPR) measuring technique, the quartz crystal microbalance (QCM) measuring technique, a technique that utilizes the functional surfaces of gold colloid particles to superfine particles, and the like.

For example, a surface plasmon resonance biosensor comprises a member having a portion that transmits and reflects light irradiated from a sensor, and a portion at which bioactive substances are immobilized. The biosensor chip of the present invention may be employed as the portion at which the bioactive substances are immobilized.

As a surface plasmon measuring apparatus that utilizes the fact that surface plasmon are excited by light waves to analyze substances, there is that employing a system called the "Kretschmann configuration" (see Japanese Unexamined Patent Publication No. 6 (1994) -167443, for example) The surface plasmon resonance sensor employing the "Kretschmann configuration" is equipped basically with a dielectric block formed, for example, into the shape of a prism; a metal film, formed on a surface of the dielectric block, for placing a sample thereon; a light source for emitting a light beam; an optical system for making the light beam enter the dielectric block at various angles of incidence so that the condition for total internal reflection is satisfied at the interface between the dielectric block and the metal film; and photodetecting means for detecting the state of the surface plasmon resonance, that is, the state of ATR, by measuring the intensity of the light beam totally internally reflected at the interface.

In addition, a leaky mode sensor is known as a similar measuring apparatus that utilizes ATR, as disclosed, for instance, in "Spectral Researches," Vol. 47, No.1 (1998), pp. 21-23 and pp. 26-27. The leaky mode sensor is constructed basically by: a dielectric block in the form of a prism, for example; a cladding layer formed on a surface of the dielectric block; an optical waveguide layer, formed on the cladding layer, for placing a sample thereon; a light source for emitting a light beam; an optical system for making the light beam enter the dielectric block at various angles of incidence so that the condition for total internal reflection is satisfied at the interface between the dielectric block and the cladding layer; and photodetecting means for detecting the excited state of the waveguide mode, that is, the state of ATR, by measuring the intensity of the light beam totally internally reflected at the interface between the dielectric block and the cladding layer. The biosensor chip of the present invention may also be employed in leaky mode measuring apparatuses.

In addition, the biosensor chip of the present invention may be employed as a chip for a biosensor having a waveguide structure constituted by a diffraction grating and additional layers as necessary on a substrate. This type of biosensor detects changes in refractive index by using the waveguide structure. Details of biosensors of this type are described at page 4, line 48 through page 14, line 15, and Figures 1 through 8 of Japanese Patent Publication No. 6(1994) -27703, and column 6, line 31 through column 7, line 47, and Figures 9A and 9B of U.S. Patent No. 6,829,073. The biosensor chip of the present invention may also be employed in a modified version of this type of biosensor, in which arrays of diffraction grating waveguides are incorporated within wells of a microplate, such as that disclosed in PCT Japanese Publication No. 2007-501432. In the case that the diffraction grating waveguides are arranged in arrays at the bottom surfaces of the wells of a microplate, screening of drugs and chemical substances is enabled with high throughput.

Embodiments of the biosensor chip of the present invention will be described hereinafter.

### [Embodiment 1]

### (Production of the biosensor chip)

A UV ozone treatment was administered to an Au sensor chip by Biacore, on which only a metal film is provided, for twelve minutes. Then, a 10ml solution containing 5µmol of C2-NTA (3,3'-dithiobis [N- (5-amino-5-carboxypentyl) propionamide-N,N'-diacetic acid] dihydrochloride) by Dojin Chemical and 5µmol of 6-hydroxy-1-hexanethiol by Aldrich in ultrapure water was prepared. The metal film was caused to react with the solution for sixteen hours at 40°C, then washed once with ethanol and once with ultrapure water, to produce the biosensor chip.

### (Immobilization of Enzymes on the Chip)

The biosensor chip produced as described above was set in Biacore 3000 by Biacore, which is a surface plasmon resonance apparatus. The biosensor chip was stabilized by an SPR HEPES buffur liquid (20mM HEPES-HCl, 150mM NaCl, pH7.2) at a flow rate of 10µl/min. 10µl of a 100µmol/l CuCl₂ solution was added. Thereafter, the biosensor chip was washed with 20µl of an HBS-N buffer. Next, 10µl of a 100nmol/l His10-GFP solution was added, and it was confirmed that proteins can be immobilized on the biosensor chip. The biosensor chip was regenerated, by washing with 10µl of a 100µmol/l imidazole solution.

### [Embodiment 2]

A biosensor chip was produced in the same manner as that of Embodiment 1, except that 1-hexanethiol by Aldrich was used instead of 6-hydroxy-1-hexanethiol.

### [Embodiment 3]

A biosensor chip was produced in the same manner as that of Embodiment 1, except that the amount of C2-NTA was changed to 3µmol, and 7µmol of 5-hydroxy-1-pentanethiol was used instead of 6-hydroxy-1-hexanethiol.

### [Embodiment 4]

A biosensor chip was produced in the same manner as that of Embodiment 1, except that a 5/5 mixture of 6-hydroxy-1-hexanethiol and 1-hexanethiol was used instead of 6-hydroxy-1-hexanethiol.

### [Embodiment 5]

A biosensor chip was produced in the same manner as that of Embodiment 1, except that 6-methoxy-1-hexanethiol by Aldrich was used instead of 6-hydroxy-1-hexanethiol.

### [Embodiment 6]

A biosensor chip was produced in the same manner as that of Embodiment 1, except that the amount of C2-NTA was changed to 7µmol and the amount of 6-hydroxy-1-hexanethiol was changed to 3µmol.

### [Comparative Example 1]

A biosensor chip was produced in the same manner as that of Embodiment 1, except that 6-hydroxy-1-hexanethiol was not used.

### (Stability Evaluations)

In order to evaluated the stability of the biosensor chips, 50µl of a 10mmol/L CuCl₂ solution was added to the surfaces of the biosensor chips at a flow rate of 10µl/min, then the biosensor chips were washed with 10µl of a 100µmol/L imidazole solution, and changes before and after the washing was measured by Biacore 3000, which is a surface plasmon resonance apparatus manufactured by Biacore.

The results are illustrated in Table 1. Note that the ratio of the number of alkyl chains to which nitrilotriacetic acid is bonded with respect to the total number of alkyl chains on the substrate is practically the mixture ratio between alkanethiol and NTA, and therefore these values are used here. Alternatively, the ratio can be calculated employing data obtained by observation with an atomic force microscope.

**TABLE 1**

| | Ligand | Self Assembled Monolayer Forming Compound | Mix Ratio | NTA Bonding Ratio | Stability Evaluation (ΔRU) |
|---|---|---|---|---|---|
| Embodiment 1 | C2-NTA | 6-Hydroxy-1-hexanethiol | 5/5 | 0.67 | -10 |
| Embodiment 2 | C2-NTA | 1-hexanethiol | 5/5 | 0.67 | -8 |
| Embodiment 3 | C2-NTA | 5-Hydroxy-1-pentanethiol | 3/7 | 0.46 | +15 |
| Embodiment 4 | C2-NTA | 6-Hydroxy-1-hexanethiol/ 1-hexanethiol 5/5 mixture | 5/5 | 0.67 | -10 |
| Embodiment 5 | C2-NTA | 6-Methoxy-1-hexanethiol | 5/5 | 0.67 | -10 |
| Embodiment 6 | C2-NTA | 6-Hydroxy-1-hexanethiol | 7/3 | 0.82 | -5 |
| Comparative Example 1 | C2-NTA | None | 10/0 | 1 | -50 |

In Embodiments 1 through 6, the ratios of the number of alkyl chains which are covalently bonded to the ligands with respect to the total number of alkyl chains on the substrate are within a range of 0.40 to 0.99. Therefore, as is clear from the results of Table 1, the change in RU prior to and following washing is small for Embodiments 1 through 6, and it can be seen that separation of the ligands from the surface of the biosensor chip due to the metal ions is suppressed.

## Claims

1. A biosensor chip, comprising:
a substrate;
a metal film which is physically attached to the substrate; and
a self assembled monolayer constituted by alkyl chains, which is physically attached to the metal film; **characterized by**:
ligands and at least one functional group, selected from a group consisting of a hydroxyl group, a carboxyl group, an alkoxy group, a methyl group, and hydrogen atoms being physically attached to the self assembled monolayer; and
the ratio of the number of alkyl chains which are covalently bonded to the ligands with respect to the total number of alkyl chains on the substrate being within a range of 0.40 to 0.99, and more preferably within a range of 0.45 to 0.85.

2. A biosensor chip as defined in Claim 1, **characterized by**:
metal ions being coordinately bonded to the ligands.

3. A biosensor chip as defined in Claim 2, **characterized by**:
bioactive substances being coordinately bonded to the metal ions.

4. A biosensor chip as defined in any one of Claims 1 through 3, **characterized by**:
the ligands being nitrilotriacetic acid derivatives.

5. A biosensor chip as defined in any one of Claims 1 through 4, **characterized by**:
the alkyl chains that constitute the self assembled monolayer being covalently bonded with the metal film by at least one functional group, selected from a group consisting of: -SH, -SS-, -SeH, -SeSe, and -COSH, or by the reaction of said functional group(s) with the metal film.

6. A biosensor chip as defined in any one of Claims 1 through 5, **characterized by**:
the self assembled monolayer being formed by a compound represented by the following General Formula (1) and a mixture that contains a compound represented by the following General Formula (2)
X-R^{a}-Y (1)
X-R^{b}-Z (2)
wherein: X represents a group which is capable of covalent bonding with the metal film; R^{a} and R^{b} represent organic divalent linking groups; Y is a functional group selected from a group consisting of: a hydroxyl group, a carboxyl group, an alkoxy group, a methyl group, and hydrogen atoms; and Z represents a ligand.

7. A biosensor chip as defined in Claim 6, **characterized by**:
the carbon number of the alkyl chain length of R^{a} being within a range from 2 to 8, and more preferably within a range from 4 to 8.

8. A biosensor chip as defined in Claim 7, **characterized by**:
the difference in carbon numbers between R^{a} and R^{b} being within a range from 2 to 6.

9. A biosensor chip as defined in any one of Claims 2 through 8, **characterized by**:
the metal ions being Cu(II) ions.

10. A biosensor chip as defined in any one of Claims 1 through 9, **characterized by**:
the metal film being formed by at least one metal selected from a group consisting of: gold, silver, copper, platinum, palladium, and aluminum.
